# EUROPEAN PATENT APPLICATION

(11) **EP 4 616 822 A1**
(43) Date of publication of application: **17.09.2025**
(21) Application number: 24163508.5
(22) Date of filing: 14.03.2024
(51) Int. Cl.: A61B 46/20, A61B 8/00, A61B 90/00

(54) **IMMERSION SYSTEM AND METHOD FOR ULTRASOUND-ASSISTED SURGERY ON A PATIENT'S UPPER OR LOWER LIMB**

(71) Applicant: SPIRECUT SA, 4132 Muttenz (CH)
(72) Inventor: MOUNGONDO, Fabian, 7340 Colfontaine (BE); SCHUIND, Frédéric, 1801 Le Mont-Pèlerin (CH)
(74) Representative: ABREMA SA

(57) **Abstract**

There is described an immersion system (100) for ultrasound-assisted surgery on a patient's upper or lower limb (LB) comprising a basin (B) configured and dimensioned to receive a part of the patient's upper or lower limb (LB), the basin (B) exhibiting a retention wall (10W) defining an accommodating space (10S) for the part of the patient's upper or lower limb (LB). The immersion system (100) further comprises at least one impermeable surgical drape (SD) that is draped over the basin (B) and exhibits an aperture (SDa) dimensioned to allow passage of the patient's upper or lower limb (LB) through the at least one impermeable surgical drape (SD) to expose a portion of the patient's upper or lower limb (LB) within an immersible space (S) formed by the accommodating space (10S) of the basin (B) draped by the at least one impermeable surgical drape (SD). The immersible space (S) is configured to allow immersion of the exposed portion of the patient's upper or lower limb (LB) in sterile liquid (L) poured within the immersible space (S).

## Description

### TECHNICAL FIELD

The present invention generally relates to an immersion system and method for ultrasound-assisted surgery on a patient's upper or lower limb.

### BACKGROUND OF THE INVENTION

Ultrasound-assisted surgery is applied to a variety of surgical procedures, including but not limited to percutaneous release procedures as taught for instance in International (PCT) Publications Nos. WO 2020/003263 A1 and WO 2023/079439 A1, the contents of which are incorporated herein by reference in their entirety. An ultrasound probe is indeed used in such case to provide suitable sonographic imagery helping the surgeon to identify the position and orientation of surgical instruments within the tissues and thereby assist the surgeon in the performance of the surgical release procedure.

Sonographic imagery may be improved and/or facilitated by immersing the relevant portion of the patient's body being probed using ultrasound. Such an approach is for instance documented in *"*Water Bath Evaluation Technique for Emergency Ultrasound of Painful Superficial Structures", Michael Blaivas et al., American Journal of Emergency Medicine, Volume 22, Number 7, November 24, pp. 589-593 (https://doi.org/10.1016/j.ajem.2004.09.009). In this case, a water bath is used to immerse part of a patient's upper or lower limb (especially a patient's hand or foot) in water to reduce patient's discomfort during ultrasound probing, doing away with the necessity to apply firm transducer contact between the ultrasound probe and the patient's skin or use of ultrasound gel to provide adequate contact with the ultrasound probe. In effect, the water bath replaces the need for ultrasound gel or contact between the ultrasound transducer and the patient's skin, water being an excellent medium for ultrasound transmission.

According to the aforementioned article, relatively rudimentary means are contemplated to allow immersion of, especially, the patient's hand or foot, namely use of a conventional bedpan or like conventional basin filled with sterile water or saline solution. These rudimentary solutions, while suitable for treating relatively minor injuries or traumas in emergency medicine, are not adequate for performing more elaborate surgical procedures, such as but not limited to percutaneous release procedures.

Furthermore, the use of bedpans or like conventional basins is at most adequate for cases where the patient is cooperative and may hold his or her hand or foot in a correct setting and where the procedure is limited to ultrasound exploration.

A further, specific example of this approach is briefly discussed in *"*Ultrasound of the Flexor Tendons of the Fingers", Thomas Apard, "Ultrasonography for the Upper Limb Surgeon", Thomas Apard and Jean Louis Brasseur (Editors), Part V, Finger, Chapter 18, pp. 169-172, Springer, January 5, 2022, ISBN: 978-3-030-84233-8 (https://doi.ora/10.1007/978-3-030-84234-5 18). This article shows a device consisting in essence of a plastic recipient equipped with longitudinal bars to position a patient's finger in a given flexed position, while being immersed in water, to perform underwater ultrasound for exploration of broken or repaired pulleys. This device is specifically and mainly designed to investigate recovery after surgical reconstruction of closed finger pulley rupture e.g. in rock climbers.

There therefore remains a need for a solution that can adequately help and assist a surgeon in performing ultrasound-assisted surgery on a patient's upper or lower limb.

### SUMMARY OF THE INVENTION

A general aim of the invention is to provide a solution to help and assist a surgeon in performing ultrasound-assisted surgery on a patient's upper or lower limb, including but not limited to percutaneous release procedures.

More specifically, an aim of the present invention is to provide such a solution that can adequately be used and implemented in a surgical environment.

Yet another aim of the invention is to provide such a solution that is reasonably easy, practical and cost-efficient to implement.

A further aim of the invention is to provide such a solution that does not negatively impact comfort for the patient.

These aims, and others, are achieved thanks to the invention defined by the appended claims.

In accordance with the solution defined in claim 1, there is provided an immersion system for ultrasound-assisted surgery on a patient's upper or lower limb comprising a basin configured and dimensioned to receive a part of the patient's upper or lower limb, the basin exhibiting a retention wall defining an accommodating space for the part of the patient's upper or lower limb. The immersion system further comprises at least one impermeable surgical drape that is draped over the basin and exhibits an aperture dimensioned to allow passage of the patient's upper or lower limb through the at least one impermeable surgical drape to expose a portion of the patient's upper or lower limb within an immersible space formed by the accommodating space of the basin draped by the at least one impermeable surgical drape. The immersible space is configured to allow immersion of the exposed portion of the patient's upper or lower limb in sterile liquid poured within the immersible space.

In accordance with a particularly preferred embodiment, the retention wall of the basin is interrupted to form a lateral opening configured and dimensioned to allow passage of the patient's upper or lower limb. Advantageously, the lateral opening exhibits a sloped leading edge into the accommodating space. Ideally, the lateral opening is configured and dimensioned, along with the at least one impermeable surgical drape and aperture formed therethrough, to maintain liquid-tightness of the immersible space.

By way of preference, the basin is made of foam material, especially a moulded self-skinning foam material exhibiting a flexible outer skin encasing a comparatively softer foam core. The basin may in particular be made of polyurethane foam.

Advantageously, the retention wall is configured to act as support for a surgeon's and/or assistant's forearms and/or hands during surgery, which allows the medical team to reduce fatigue during the surgical procedure and adequately focus on the surgical act.

In accordance with a preferred embodiment of the invention, the at least one impermeable surgical drape includes a pair of superimposed surgical drapes each provided with said aperture, which helps ensuring liquid-tightness of the immersible space.

If necessary, the immersion system may further comprise a non-constricting elastic band or gluing tape configured and dimensioned to be positioned around the patient's upper or lower limb and to maintain a liquid-tight connection between the at least one impermeable surgical drape and the patient's upper or lower limb.

There is also provided a method of immersing a patient's upper or lower limb in sterile liquid, comprising the following steps:
- providing a basin configured and dimensioned to receive a part of the patient's upper or lower limb, the basin exhibiting a retention wall defining an accommodating space for the part of the patient's upper or lower limb;
- providing at least one impermeable surgical drape exhibiting an aperture dimensioned to allow passage of the patient's upper or lower limb through the at least one impermeable surgical drape;
- passing the part of the patient's upper or lower limb through the aperture formed in the at least one impermeable surgical drape and draping the at least one impermeable surgical drape over the basin and the accommodating space thereof to form an immersible space and expose a portion of the patient's upper or lower limb within the immersible space; and
- pouring sterile liquid in the immersible space to cause immersion of the exposed portion of the patient's upper or lower limb.

By way of preference, the retention wall of the basin is interrupted to form a lateral opening configured and dimensioned to allow passage of the patient's upper or lower limb, and the method further comprises positioning the patient's upper or lower limb across the lateral opening.

In the context of this method, the basin may likewise be made of a foam material, such as polyurethane foam, especially a moulded self-skinning foam material exhibiting a flexible outer skin encasing a comparatively softer foam core.

Similarly, providing the at least one impermeable surgical drape may include providing a pair of superimposed surgical drapes each provided with said aperture, which helps ensuring liquid-tightness of the immersible space.

Further advantageous embodiments of the invention are discussed below.

### BRIEF DESCRIPTION OF THE DRAWINGS

Other features and advantages of the present invention will appear more clearly from reading the following detailed description of embodiments of the invention which are presented solely by way of non-restrictive examples and illustrated by the attached drawings in which:
Figure 1A is a schematic partial perspective view of an immersion system, in accordance with a preferred embodiment of the invention, showing a basin thereof and part of a patient's upper limb positioned within an accommodating space of the basin;
Figure 1B is a schematic perspective view of the immersion system of Figure 1A with the basin draped by a pair of superimposed impermeable surgical drapes each provided with an aperture to allow passage of the patient's upper limb to expose a portion thereof within an immersible space;
Figure 1C is a schematic perspective view of the immersion system of Figure 1B where the immersible space is filled with sterile liquid to immerse the exposed portion of the patient's upper limb;
Figure 2A is a first perspective view of a preferred embodiment of the basin;
Figure 2B is a second perspective view of the basin of Figure 2A shown from a different point of view;
Figure 2C is a cross-sectional view of the basin of Figures 2A-B taken longitudinally through the basin along a vertical sectional plane passing through a lateral opening of the basin;
Figure 3A is a photographic illustration of an implementation of the immersion system of the invention in a surgical environment, with a portion of a patient's left arm being exposed within the immersible space of the immersion system;
Figure 3B is another photographic illustration of the implementation of the immersion system of Figure 3A shown from a different point of view; and
Figure 3C is a photographic illustration showing a surgeon in the process of using an ultrasound probe along part of the exposed portion of the patient's left hand.

### DETAILED DESCRIPTION OF EMBODIMENTS OF THE INVENTION

The present invention will be described in relation to various illustrative embodiments. It shall be understood that the scope of the invention encompasses all combinations and sub-combinations of the features of the immersion system disclosed herein as defined by the appended claims.

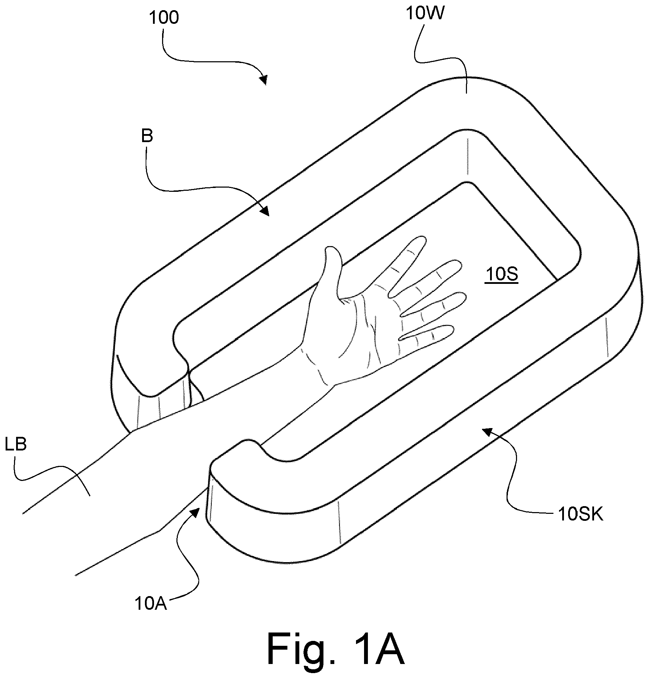

Figure 1A is a schematic partial perspective view of an immersion system, designated globally by reference numeral 100, in accordance with a preferred embodiment of the invention. Only part of the immersion system 100 is depicted schematically in Figure 1A, namely a basin B thereof, as well as part of a patient's upper limb LB (namely part of the patient's left arm) that is positioned within an accommodating space 10S of the basin B. In the illustration of Figure 1A, the basin B is configured and dimensioned to receive an entirety of the patient's left hand, as well as part of the patient's forearm. The configuration (including the shape) and/or dimensions of the basin B may obviously vary depending on the relevant part of the patient's upper or lower limb to be accommodated.

More specifically, in the illustrated example, the basin B exhibits a retention wall 10W defining the accommodating space 10S for the part of the patient's left arm LB, the retention wall 10W being here advantageously interrupted to form a lateral opening 10A that is configured and dimensioned to allow passage of the patient's forearm, as shown.

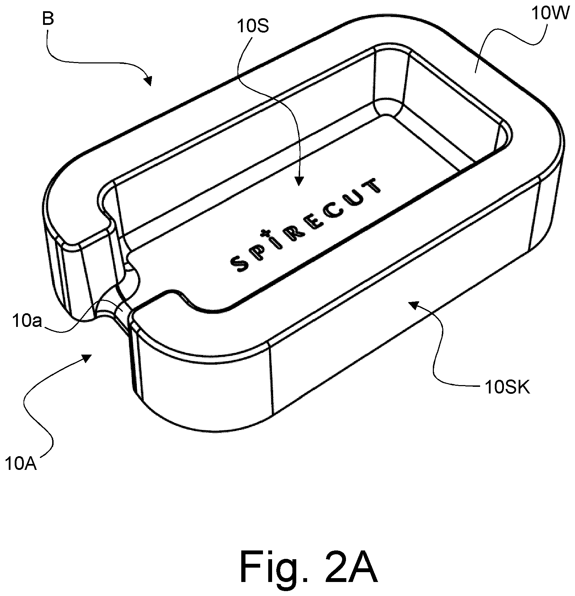

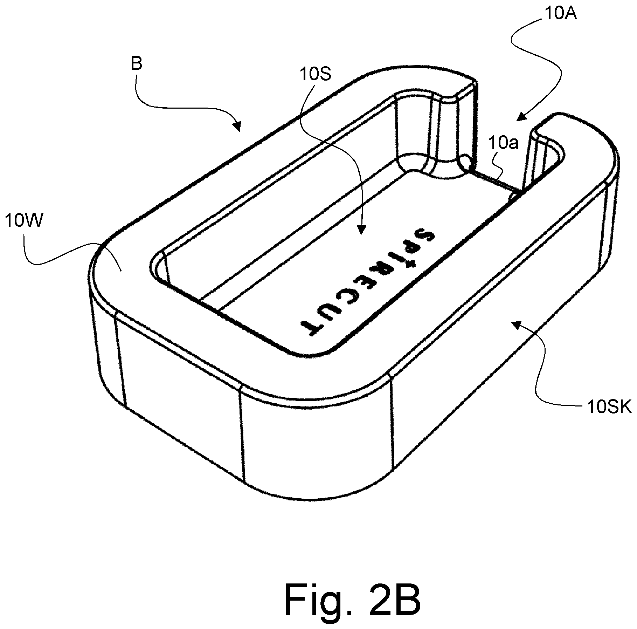

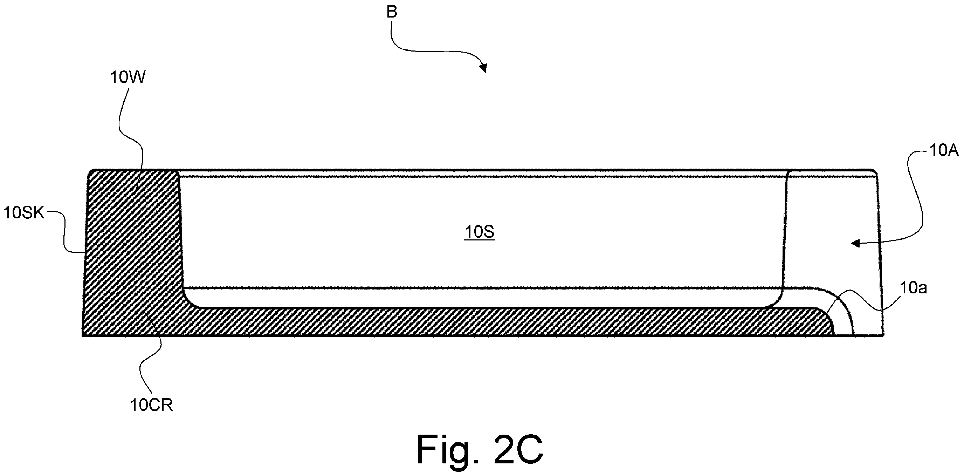

The basin B is further shown in isolation in Figures 2A to 2C. By way of preference, the lateral opening 10A exhibits a sloped leading edge 10a into the accommodating space 10S to improve comfort for the patient.

The basin B may be made of any adequate material. The basin B may especially be made of foam material, such as polyurethane foam. In accordance with a particularly preferred embodiment, the basin B is made of a moulded self-skinning foam material exhibiting a flexible outer skin 10SK encasing a comparatively softer foam core 10CR. Self-skinning foam materials are known as such in the art and have the advantage of allowing the formation of the flexible outer skin 10SK, encasing the soft foam core 10CR, in a single moulding process, using a single type of foam material. This provides some level of structural resistance and integrity to the basin B, ensuring that the basin B retains a desired shape, while leading to a relatively soft structure that is comfortable both for the patient and the surgeon and/or assistant. In effect, the retention wall 10W can be configured to act as support for the surgeon's and/or assistant's forearms and/or hands during surgery.

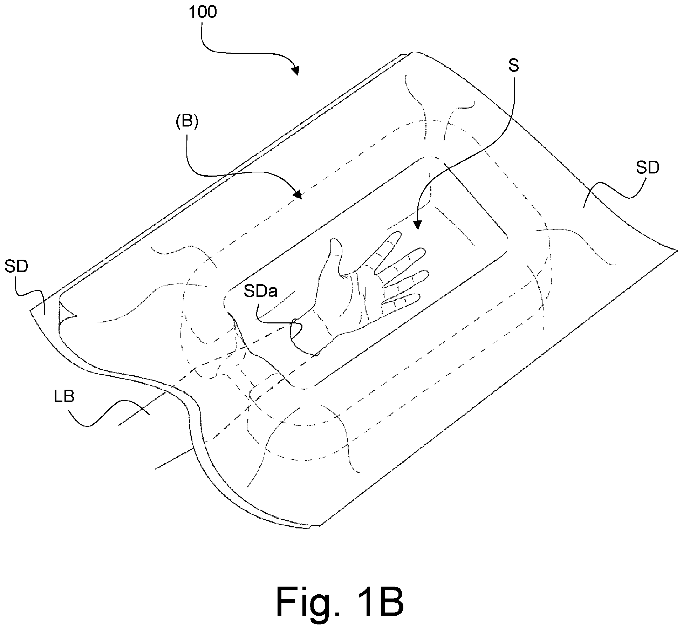

Figure 1B is a schematic perspective view of the immersion system 100 of Figure 1A where the basin B is draped by a pair of superimposed impermeable, sterile surgical drapes SD, each provided with an aperture SDa to allow passage of the patient's disinfected upper limb LB to expose a portion thereof, namely the patient's left hand in the illustrated example, within an immersible space S that is formed by the accommodating space 10S of the basin B draped by the surgical drapes SD. Each aperture SDa is preferably formed so as to conform to the size of the relevant portion of the limb LB and such that the surrounding portion of the surgical drapes SD (which inherently exhibits a certain elasticity) maintains a liquid-tight connection with the relevant portion of the limb LB.

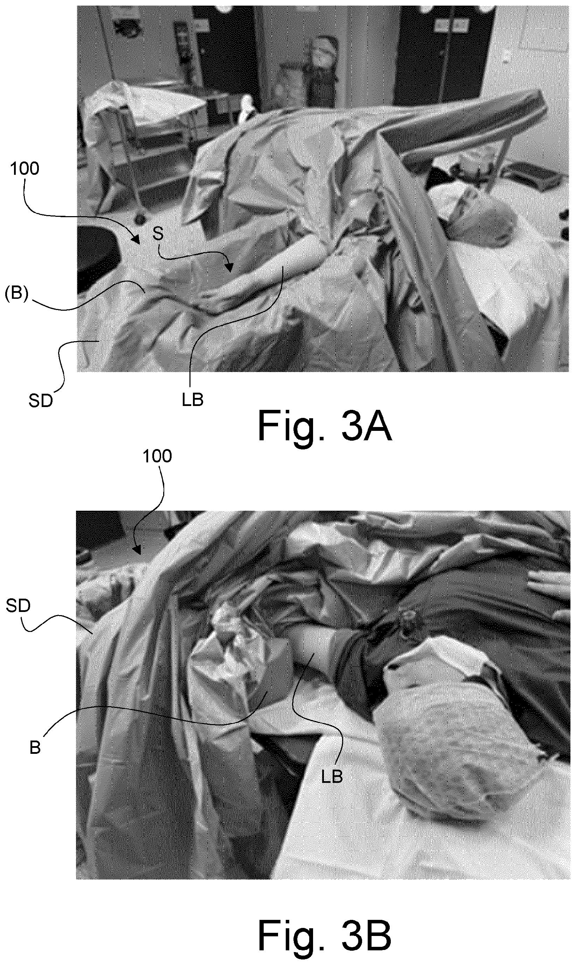

The photographic illustrations of Figures 3A-C show a possible implementation of the immersion system 100 of the invention, similar to that depicted in Figures 1A-C and 2A-C, in a surgical environment, with a portion of a patient's left arm LB being exposed within the immersible space S of the immersion system 100.

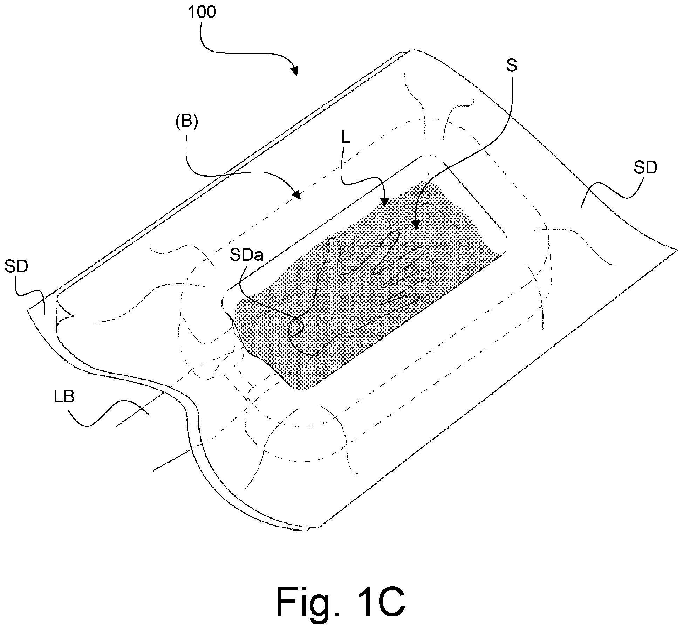

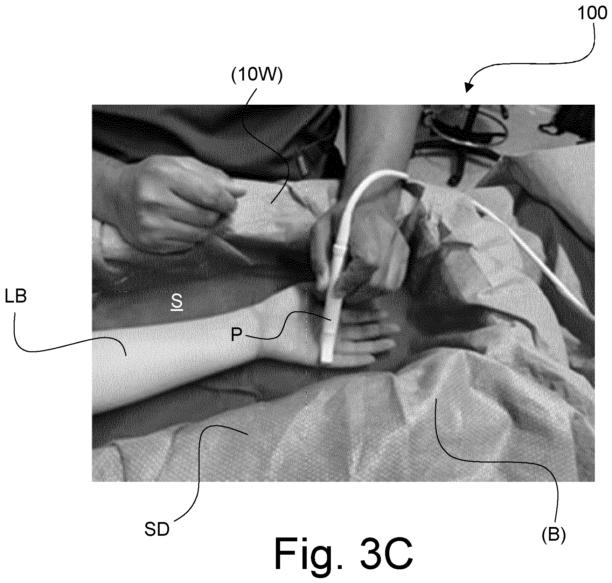

Once set up in accordance with the arrangement shown in Figure 1B, the immersible space S may be filled with sterile liquid L (such as a sterile saline solution) to immerse the exposed portion of the patient's limb LB, as shown schematically in Figure 1C. In this way, the immersion system 100 with its immersible space S, filled with sterile liquid L, provides a favourable environment and setting to use an ultrasound probe P (as shown e.g. in Figure 3C) on the exposed portion of the patient's limb LB, leading to improvements in the resulting sonographic imagery, without there being a need for a contact between the ultrasound probe P and the patient's skin as the sterile liquid L acts a suitable medium for ultrasound transmission. This liquid L may advantageously be and maintained lukewarm for the comfort of the patient, particularly if the surgical procedure happens to last long.

The lateral opening 10A formed in the retention wall 10W may advantageously be configured and dimensioned, along with the impermeable surgical drapes SD and aperture SDa formed therethrough, to maintain liquid-tightness of the immersible space S. A pair of surgical drapes SD has been found to be sufficient to adequately prevent leaks, but additional measures could be taken to ensure appropriate liquid-tightness of the immersible space S, especially in the region of the apertures SDa formed in the surgical drapes SD. For instance, the immersion system 100 may further comprise a non-constricting elastic band or gluing tape configured and dimensioned to be positioned around the patient's upper (or lower) limb LB and to maintain a liquid-tight connection between the impermeable surgical drapes SD and the patient's upper or lower limb LB. In that regard, a single impermeable surgical drape SD may also happen to be sufficient.

As mentioned above, and as emphasized in Figure 3C, the retaining wall 10W of the basin B may advantageously act as support for the surgeon's (and/or assistant's) forearms and/or hands during surgery. The surgeon may for instance rest one hand holding the ultrasound probe P on the retaining wall 10W, while using the other hand to carry out further tasks, including but not limited to performing a percutaneous release procedure on the patient's hand, using e.g. the surgical instruments and procedures described in International (PCT) Publications Nos. WO 2020/003263 A1 and WO 2023/079439 A1.

Various modifications and/or improvements may be made to the above-described embodiments without departing from the scope of the invention as defined by the appended claims.

In particular, while a pair of impermeable surgical drapes may be contemplated for draping the basin, use of a single impermeable surgical drape may be envisaged, or even more than two surgical drapes, if desired or necessary.

Furthermore, while the basin preferably exhibits a lateral opening formed along a portion of the retention wall, other embodiments could do without such a lateral opening or with an opening shaped and dimensioned to conform to and/or support the patient's limb. A lateral opening is preferred in that the basin can be positioned next to the patient so that the bottom surface of the accommodating space of the basin is close to the surface over which the patient is positioned during surgery, reducing as much as possible discomfort for the patient and ensuring that the patient's limb rests in a substantially horizontal and relaxed position.

One will also appreciate that the invention is applicable to sterile immersion of part of any one of the patient's upper and lower limbs, be it the hand or foot or part or all of the arm or leg.

It will also be appreciated that any adequate shape could be imparted to the basin. For instance, the basin may exhibit a more complex shape and configuration conforming to the relevant portion of the upper or lower limb to be treated.

### LIST OF REFERENCE NUMERALS AND SIGNS USED THEREIN

- 100: immersion system
- LB: patient's upper (or lower) limb
- B: basin made e.g. of moulded self-skinning polyurethane foam material
- 10W: retention wall of basin B
- 10A: lateral opening formed along retention wall 10W
- 10a: sloped leading edge of lateral opening 10A
- 10S: accommodating space of basin B configured and dimensioned to receive part of patient's upper or lower limb LB
- 10SK: flexible outer skin of foam material
- 10CR: soft foam core of foam material encased by flexible outer skin 10SK
- SD: impermeable surgical drape(s) (sterile)
- SDa: aperture formed through impermeable surgical drape(s) SD
- S: immersible space
- L: sterile liquid (e.g. saline solution) poured in immersible space
- P: ultrasound probe

## Claims

1. An immersion system (100) for ultrasound-assisted surgery on a patient's upper or lower limb (LB) comprising a basin (B) configured and dimensioned to receive a part of the patient's upper or lower limb (LB), the basin (B) exhibiting a retention wall (10W) defining an accommodating space (10S) for the part of the patient's upper or lower limb (LB),
wherein the immersion system (100) further comprises at least one impermeable surgical drape (SD) that is draped over the basin (B) and exhibits an aperture (SDa) dimensioned to allow passage of the patient's upper or lower limb (LB) through the at least one impermeable surgical drape (SD) to expose a portion of the patient's upper or lower limb (LB) within an immersible space (S) formed by the accommodating space (10S) of the basin (B) draped by the at least one impermeable surgical drape (SD),
and wherein the immersible space (S) is configured to allow immersion of the exposed portion of the patient's upper or lower limb (LB) in sterile liquid (L) poured within the immersible space (S).

2. The immersion system (100) according to claim 1, wherein the retention wall (10W) of the basin (B) is interrupted to form a lateral opening (10A) configured and dimensioned to allow passage of the patient's upper or lower limb (L).

3. The immersion system (100) according to claim 2, wherein the lateral opening (10A) exhibits a sloped leading edge (10a) into the accommodating space (10S).

4. The immersion system (100) according to claim 2 or 3, wherein the lateral opening (10A) is configured and dimensioned, along with the at least one impermeable surgical drape (SD) and aperture (SDa) formed therethrough, to maintain liquid-tightness of the immersible space (S).

5. The immersion system (100) according to any one of the preceding claims, wherein the basin (B) is made of foam material.

6. The immersion system (100) according to claim 5, wherein the basin (B) is made of a moulded self-skinning foam material exhibiting a flexible outer skin (10SK) encasing a comparatively softer foam core (10CR).

7. The immersion system (100) according to claim 5 or 6, wherein the basin (B) is made of polyurethane foam.

8. The immersion system (100) according to any one of the preceding claims, wherein the retention wall (10W) is configured to act as support for a surgeon's and/or assistant's forearms and/or hands during surgery.

9. The immersion system (100) according to any one of the preceding claims, wherein the at least one impermeable surgical drape (SD) includes a pair of superimposed surgical drapes (SD) each provided with said aperture (SDa).

10. The immersion system (100) according to any one of the preceding claims, further comprising a non-constricting elastic band or gluing tape configured and dimensioned to be positioned around the patient's upper or lower limb (LB) and to maintain a liquid-tight connection between the at least one impermeable surgical drape (SD) and the patient's upper or lower limb (LB).

11. A method of immersing a patient's upper or lower limb (LB) in sterile liquid (L), comprising the following steps:
- providing a basin (B) configured and dimensioned to receive a part of the patient's upper or lower limb (LB), the basin (B) exhibiting a retention wall (10W) defining an accommodating space (10S) for the part of the patient's upper or lower limb (LB);
- providing at least one impermeable surgical drape (SD) exhibiting an aperture (SDa) dimensioned to allow passage of the patient's upper or lower limb (LB) through the at least one impermeable surgical drape (SD);
- passing the part of the patient's upper or lower limb (LB) through the aperture (SDa) formed in the at least one impermeable surgical drape (SD) and draping the at least one impermeable surgical drape (SD) over the basin (B) and the accommodating space (10S) thereof to form an immersible space (S) and expose a portion of the patient's upper or lower limb (LB) within the immersible space (S); and
- pouring sterile liquid (L) in the immersible space (S) to cause immersion of the exposed portion of the patient's upper or lower limb (LB).

12. The method of claim 11, wherein the retention wall (10W) of the basin (B) is interrupted to form a lateral opening (10A) configured and dimensioned to allow passage of the patient's upper or lower limb (L),
and wherein the method further comprises positioning the patient's upper or lower limb (LB) across the lateral opening (10A).

13. The method of claim 11 or 12, wherein the basin (B) is made of foam material, such as polyurethane foam.

14. The method of claim 13, wherein the basin (B) is made of a moulded self-skinning foam material exhibiting a flexible outer skin (10SK) encasing a comparatively softer foam core (10CR).

15. The method of any one of claims 11 to 14, wherein providing the at least one impermeable surgical drape (SD) includes providing a pair of superimposed surgical drapes (SD) each provided with said aperture (SDa).
